Europäisches Patentamt

(19) European Patent Office

Office européen des brevets

(11) Numéro de publication : **0 240 430 B1**

(12)

# FASCICULE DE BREVET EUROPEEN

(45) Date de publication du fascicule du brevet :
**19.06.91 Bulletin 91/25**

(51) Int. Cl.⁵ : **A61G 13/00, A61F 5/04**

(21) Numéro de dépôt : **87400722.2**

(22) Date de dépôt : **02.04.87**

(54) **Dispositif de traction pour tables d'opérations et appareillages de chirurgie orthopédique.**

(30) Priorité : **03.04.86 FR 8604786**

(43) Date de publication de la demande :
**07.10.87 Bulletin 87/41**

(45) Mention de la délivrance du brevet :
**19.06.91 Bulletin 91/25**

(84) Etats contractants désignés :
**CH DE ES FR GB IT LI SE**

(56) Documents cités :
**DE-A- 3 241 029**
**FR-A- 1 427 080**

(73) Titulaire : **MARZET-AUBRY S.A.**
**9 rue Président Allende**
**F-94250 Gentilly (FR)**

(72) Inventeur : **Deprez, Roland**
**67 à 77 Rue Charles Frerot**
**F-94250 Gentilly (FR)**

(74) Mandataire : **Bertrand, Didier et al**
**Cabinet Beau de Loménie 55, rue**
**d'Amsterdam**
**F-75008 Paris (FR)**

## Description

La présente invention concerne les dispositifs de traction pour tables d'opérations et appareillages de chirurgie orthopédique, selon le préambule de la revendication 1. Le document FR-A-1 427 080 décrit un dispositif de ce type.

La chirurgie orthopédique est souvent pratiquée sur des tables d'opérations comportant des systèmes de traction, principalement utilisés pour des interventions sur les membres inférieurs d'un patient, qui se composent de bras de traction comportant des tracteurs mécaniques à vis. Ces bras de traction sont fixés sur un bâti qui comporte les éléments nécessaires au soutien du bassin du patient. Ces bras de traction peuvent être articulés horizontalement et verticalement.

La chirurgie orthopédique utilise de nos jours le contrôle radiologique per-opératoire d'une façon intensive. Les appareils de contrôle à source radiogène, notamment les amplificateurs de luminance nécessitent un accès facile du champ opératoire.

Les systèmes d'articulation des dispositifs de traction orthopédique, généralement volumineux et situés dans l'axe du champ opératoire, empêchent une utilisation maximale des appareils à source radiogène. Par ailleurs, les dispositifs de traction conçus pour une utilisation maximale des appareils à source radiogène sont peu maniables et leurs bras de traction ne sont que partiellement articulés.

Par exemple, on connaît par le document FR-A-2518400 un dispositif comportant un bâti ; deux bras latéraux articulés sur le bâti de manière à être mobiles horizontalement, les bras étant dotés de moyens de blocage dans leur position et des moyens de traction; et un support pelvien lié au bâti et réglable latéralement par rapport au plan médian du bâti. Dans ce dispositif, l'articulation des bras ne permet que leur déplacement horizontal, ce qui est très insuffisant pour répondre à tous les besoins de la chirurgie orthopédique. De plus, les moyens de blocage des bras, réalisés sous forme de leviers de serrage disposés au niveau des joints articulés, sont difficilement accessibles lorsque le champ opératoire est déjà encombré par des appareils annexes.

On connaît par ailleurs par le document FR-A-1 427 080 un appareillage orthopédique adaptable sur une table d'opération, selon lequel deux bras articulés latéraux sont déportés de façon à dégager le champ de contrôle radiologique, et coopèrent avec des barres de contre-extension coudés reliés à un support pelvien. La forme des barres coudées doit cependant être adaptée aux zones devant être dégagées pour le contrôle radiologique (col du fémur, bassin) et la mise en oeuvre de bras articulés latéraux présentant les mêmes performances pour chacun des membres rend la réalisation assez complexe.

Le but de l'invention est de proposer un dispositif ayant une maniabilité des bras de traction suffisante, tout en permettant une utilisation maximale des appareils de contrôle à source radiogène, grâce à un dégagement maximum du champ opératoire.

Ce but est atteint par l'invention grâce à une conception nouvelle selon les caractéristiques de la seconde partie de la revendication 1.

Cette conception a été rendue possible en prenant en compte le fait que dans la pratique, le chirurgien ne doit opérer au cours d'une même intervention qu'un seul membre du patient : il est donc possible de réserver pour ce seul membre un bras unique doté d'une articulation dans tous les sens, et de prévoir pour le membre non opéré un simple bras de contre-extension, ne nécessitant qu'une articulation simplifiée.

Avantageusement, le moyen de blocage du bras central comporte un embout de serrage coaxial au bras et manoeuvrable en rotation, traversant des lumières méridiennes croisées de deux fuseaux sphériques articulés sur des axes orthogonaux, l'embout comportant deux filetages inversés coopérant avec des mors mobiles en translation sur l'embout et situés de part et d'autre du croisement des fuseaux de manière à entraîner par leur rapprochement ou leur éloignement mutuel, le blocage par friction ou le déblocage du bras.

Avantageusement, un patin de contact est interposé entre les fuseaux au niveau de leur croisement et l'embout de serrage est manoeuvrable grâce à la rotation d'une poignée, située en bout de bras, transmise par un arbre de commande.

Ces dispositions permettent de réaliser l'articulation et le dispositif de blocage sous une forme très compacte, libérant complètement le champ opératoire.

Avantageusement, le bras de traction comporte un élément tubulaire associé à des bielles et biellettes de manière à former des parallélogrammes articulés maintenant en permanence le parallélisme avec le bras du support tracteur des moyens de traction. Du fait que, selon l'invention, la jambe du patient ne se trouve plus dans l'axe du bras de traction et donc que lesdits parallélogrammes déformables ne sont plus dans un plan vertical parallèle à la jambe du patient, la distance entre le point d'accrochage du tracteur sur le pied et la hanche du patient varie en fonction du déplacement du bras. Il est alors avantageux d'utiliser en combinaison avec ce tracteur un contrôleur électronique de tension maintenant constante la force de traction, quelle que soit la manipulation du bras. On peut se référer sur ce sujet au document FR-A-2 578 418, déposée au nom du Demandeur.

Avantageusement, le support pelvien est ajustable latéralement, grâce à un pivot lié au bâti et un système de crantage.

D'autres avantages et caractéristiques ressortiront de la description suivante d'un mode préféré de réalisation.

Il sera fait référence aux dessins annexés sur lesquels :

• la figure 1 représente une vue latérale du dispositif suivant l'invention ;

• la figure 2 représente une vue en plan du dispositif de la figure 1 ; sur cette figure, la position du bras de traction indique que l'opération est effectuée sur la jambe droite ;

• la figure 3 représente une vue en plan du dispositif de la figure 1 ; sur cette figure, la position du bras de traction indique que l'opération est effectuée sur la jambe gauche ;

• la figure 4 représente une vue latérale du dispositif, le brss de traction étant représenté en position "haute" ;

• la figure 5 représente une vue latérale du dispositif, le bras de traction étant représenté en position "basse" ;

• la figure 6 représente une vue latérale de la rotule d'articulation du bras principal ;

• la figure 7 représente une vue en plan de la rotule d'articulation du bras principal ;

• la figure 8 représente une vue latérale de l'ensemble support tracteur ;

• la figure 9 représente une vue en plan de l'ensemble support tracteur ;

• la figure 10 représente une vue en plan du support pelvien crantable ; sur cette figure, le pelvis est excentré vers la droite ;

• la figure 11 représente une vue latérale du support pelvien en position débrayée ;

• la figure 12 représente une vue en plan du support pelvien crantable ; sur cette figure, le pelvis est excentré vers la gauche.

## 1. Description générale du dispositif de l'invention

Le dispositif de l'invention comporte un bâti compact 1, solidaire par une extrémité 2 d'une table orthopédique autonome, ou bien pouvant être connectée à une table par des moyens appropriés.

Une autre extrémité 3 du bâti 1 forme une cavité sensiblement parallélépipédique destinée à loger l'articulation du bras central de traction 4.

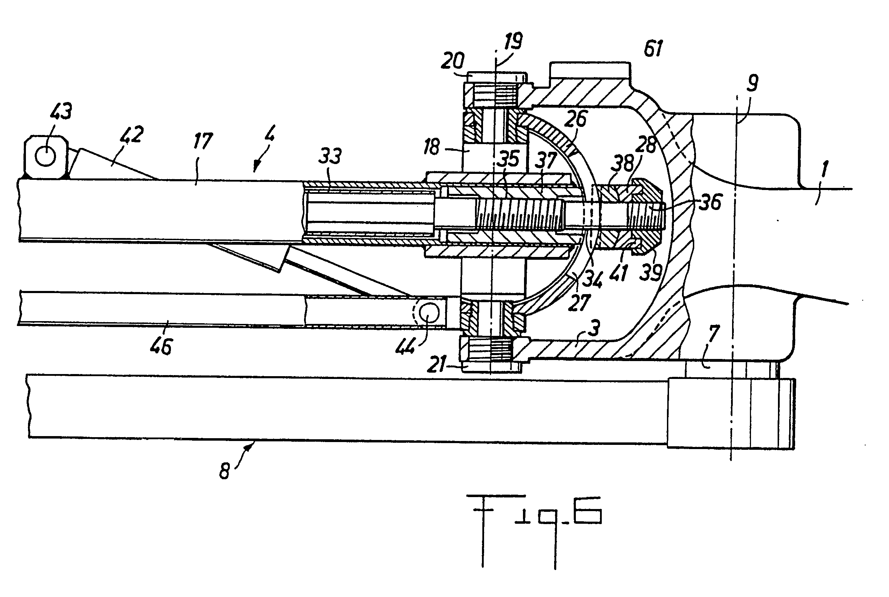

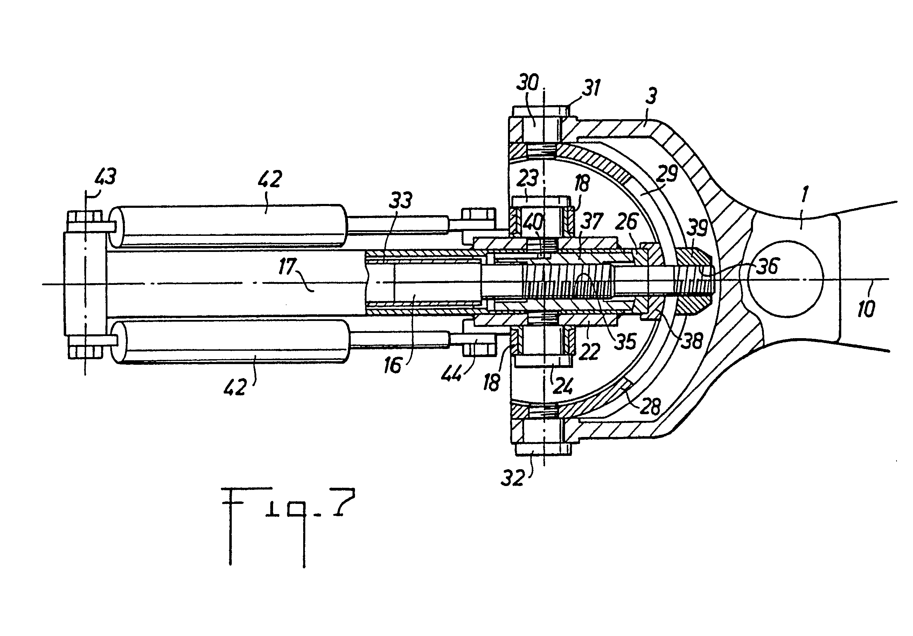

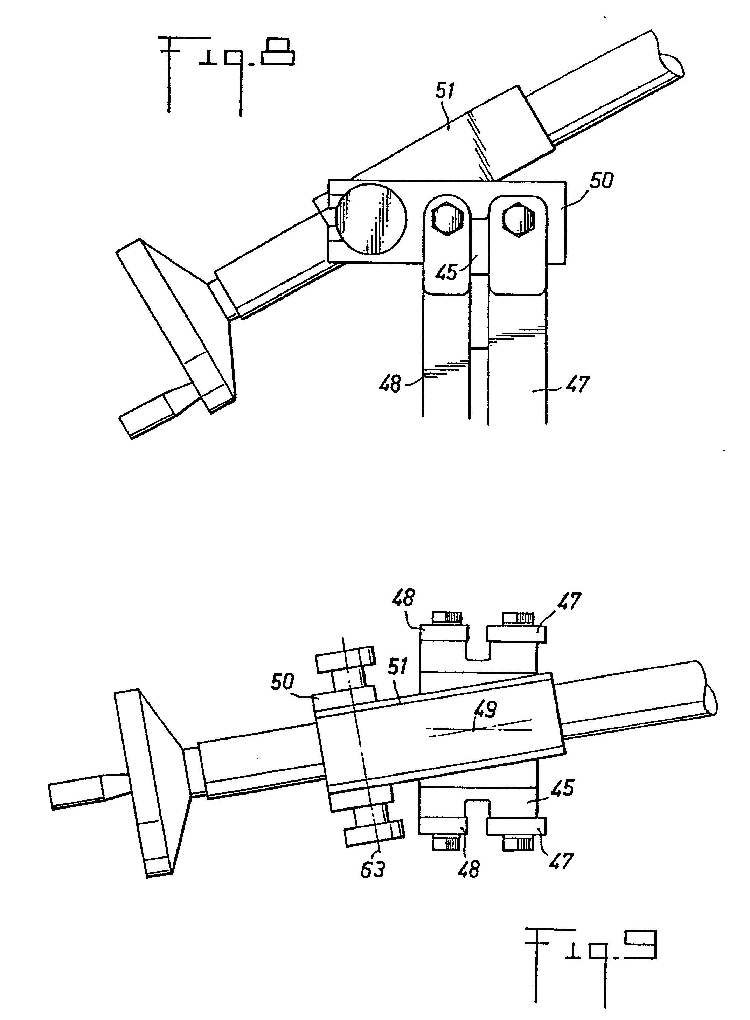

La partie médiane du bâti 1 comporte des paliers dans lesquels tourillonnent un pivot vertical supérieur 5 destiné au support pelvien 6 et un pivot vertical inférieur 7 (figure 6) gui réalise l'articulation du bras central de contre-extension 8. Les pivots 5 et 7 sont sur un même axe 9 vertical contenu dans le plan vertical médian 10 du bâti 1.

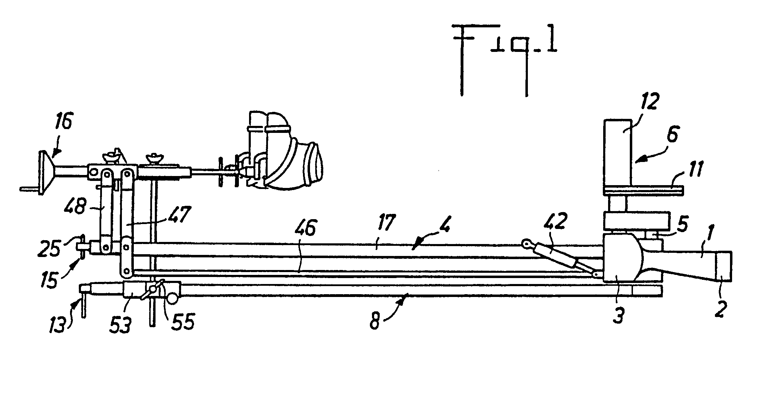

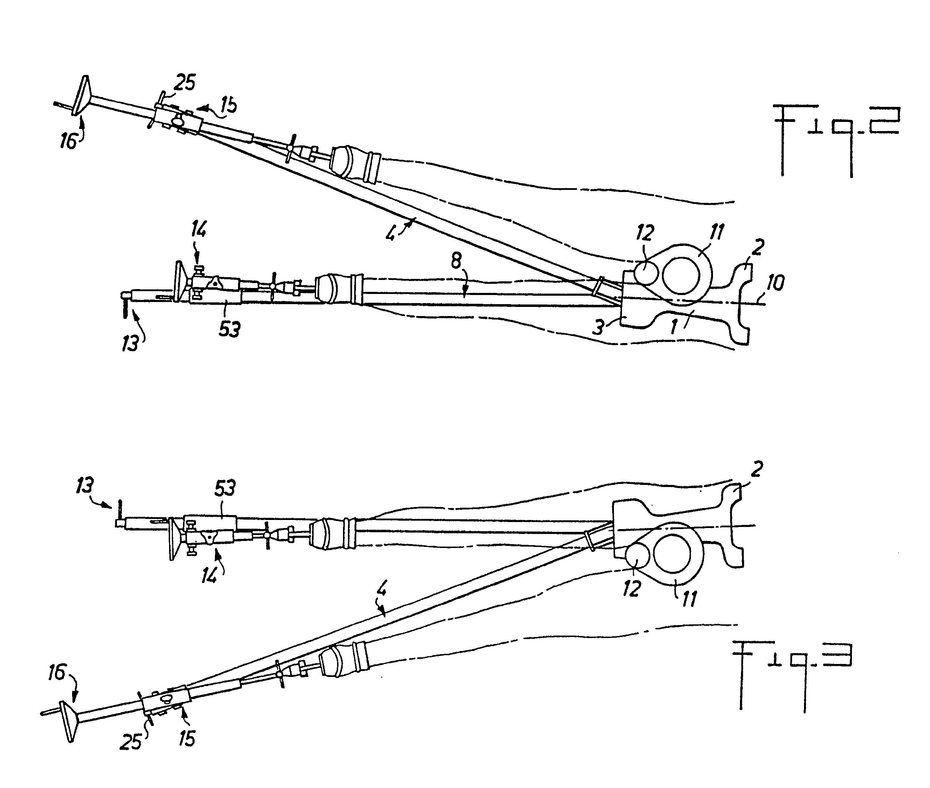

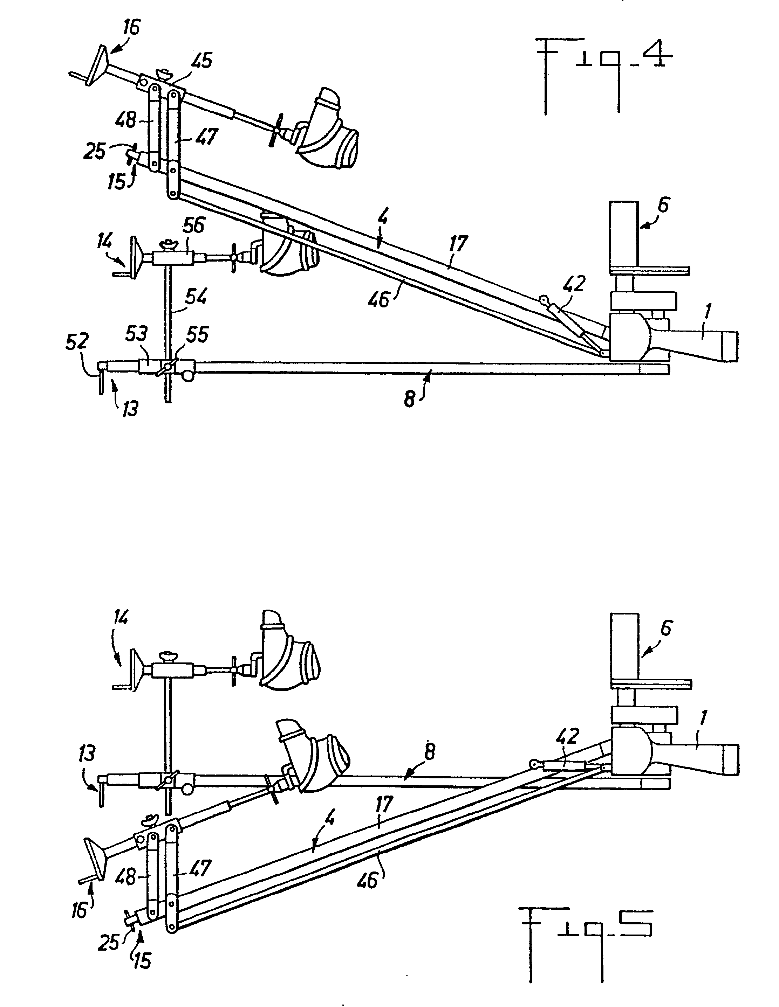

Le support pelvien 6 est monté sur le pivot 5 par des moyens qui seront détaillés plus loin, de manière que sa partie de réception, comprenant la couronne 11 et la colonne 12, puisse être placée de part ou d'autre du plan médian 10 du bâti (cf. figures 2 et 3).

Le bras de contre-extension 8 est mobile, grâce à son articulation sur le pivot 7, dans un plan horizontal. Il peut être bloqué grâce à des moyens 13 et comporte à son extrémité opposée à l'articulation des moyens de traction 14.

L'articulation du bras central de traction 4, logée dans la cavité 3 du bâti, est réalisée par un cardan centré dans le plan médian de manière à permettre au bras 4 des mouvements dans toutes les directions. Le bras 4 peut être bloqué en position quelconque par des moyens 15 et comporte également des moyens de traction 16.

L'articulation du bras central de traction 4 est située légèrement au dessus du plan horizontal balayé par le bras central de contre-extension 8.

## 2. Principe de fonctionnement du dispositif

Le bras de traction central 4, destiné à la jambe à opérer, peut recevoir indifféremment la jambe droite (figure 2) ou la jambe gauche (figure 3) du patient. Le réglage latéral du support pelvien 6 facilite l'excentrement de la jambe à opérer par rapport au plan médian 10. La jambe qui n'est pas à opérer est fixée sur le bras de contre-extension 8.

Les moyens de blocage respectifs 13 et 15, et les moyens de traction 14 et 16 permettent de choisir la position et la traction voulues sur les jambes.

La bilatéralité du dispositif est obtenue en faisant permuter le bras de traction 4 et le bras de contre-extension 8. Compte tenu de l'encombrement des extrémités des bras 4 et 8, dû aux moyens de blocage 13 et 15 et de traction 14 et 16, cette permutation s'opère en soulevant le bras 4 (figure 4) suffisamment pour qu'il passe, lors de son pivotement, au dessus des moyens de traction 14 du bras 8.

## 3. Description détaillée des différents organes

### 3.1 Le bras de traction central

#### 3.1.1. L'articulation du bras de traction central

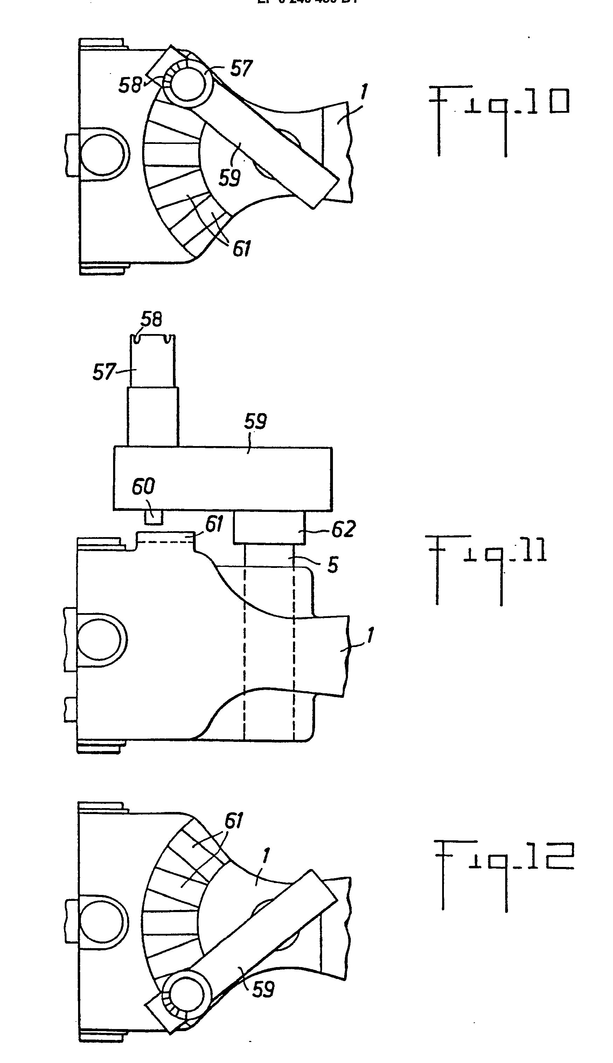

On se réfère aux figures 6 et 7.

Le bras 4 comporte un élément principal tubulaire 17 articulé sur un croisillon 18. Le croisillon 18 pivote verticalement autour de l'axe 19 grâce à des tourillons 20, 21 logés dans les parois supérieure et inférieure de la cavité 3 du bâti 1.

L'extrémité 22 de l'élément 17 porte des tourillons latéraux 23, 24 qui lui permettent de pivoter horizontalement dans les bras latéraux du croisillon 18.

L'ensemble constitue un joint de cardan.

#### 3.1.2. Les moyens de blocage du bras de traction

Une des originalités de l'invention réside dans la conception des moyens de blocage compatibles avec le cardan décrit ci-dessus.

Ces moyens, manoeuvrables par la poignée 25 située à l'autre extrémité du bras, sont constitués comme suit.

Un premier fuseau sphérique vertical 26, ou plus exactement une bande en demi grand cercle de sphère, comportant une lumière méridienne 27, est en pivotement libre autour de l'axe 19 (par l'intermédiaire des tourillons 20, 21 servant au montage du croisillon 18) ;

Un second fuseau sphérique 28, orthogonal et de rayon supérieur au premier, et comportant une lumière méridienne 29, est monté en pivotement libre autour de l'axe horizontal 31, orthogonal au plan médian 10, par l'intermédiaire de tourillons 31, 32 traversant les parois latérales de la cavité 3.

L'élément tubulaire 17 du bras 4 loge un arbre de commande 33, monté en rotation et entraîné par la poignée de serrage 25. L'arbre 33 se prolonge du côté de l'articulation du bras par un embout de serrage 34 comportant deux parties filetées de pas contraire 35, 36.

L'embout 34 traverse les lumières méridiennes orthogonales 27 et 29, de sorte que la première partie filetée 35, située du côté intérieur des fuseaux 26 et 28, coopère en translation avec un mors mobile intérieur 37, et que la seconde partie filetée 36, située du côté extérieur desdits fuseaux, coopère en translation avec un mors mobile extérieur 39. Un patin de contact 38, traversé par l'embout de serrage 34, sert d'entretoise entre les deux fuseaux 26 et 28. Une goupille 40 bloque en rotation le mors intérieur 37, tandis que des nervures 41 du fuseau 28 bloque en rotation le mors extérieur 39 doté de rainures correspondantes.

Le mécanisme de blocage est le suivant.

Une rotation de l'embout de serrage 34, provoquée sur la poignée 25 et transmise par l'arbre de commande 33, entraîne une translation inversée de chacun des mors 37, 39. Leur rapprochement des fuseaux 26, 28 provoque le serrage, leur éloignement le desserrage.

Quand les mors sont desserrés, le pivotement des fuseaux et le guidage de l'embout de serrage le long des lumières méridiennes, permettent au mécanisme de ne pas entraver la liberté de mouvement du bras 4, pour autant que l'extension angulaire maximale permise par les lumières ne soit pas atteinte.

### 3.1.3. Compensation pneumatique du bras de traction

Lorsque le bras 4 est débloqué, la seule force qui s'exerce sur lui est celle qui résulte de son poids propre et qui sollicite le bras vers le bas.

Afin d'obtenir le maintien du bras 4 en position desserrée, sans aucune intervention extérieure à l'appareillage, on prévoit un système de compensation pneumatique. Ce système est constitué de deux vérins 42, situés de part et d'autre du bras 4, articulés autour d'un pivot horizontal 43 sur l'élément tubulaire 17 et d'un pivot horizontal 44 lié au croisillon 18, de sorte que les vérins 42 puissent suivre tous les mouvements de l'élément 17.

### 3.1.4 Moyens de traction du bras de traction

Les moyens de traction 16 comportent un support tracteur 45 maintenu parallèle au bras 4 par un mécanisme articulé.

Ce mécanisme comporte d'une part une bielle 46 articulée sur le pivot 44 lié au croisillon 18 et sur une extrémité d'une biellette 47 articulée sur l'élément tubulaire 17, de manière à composer un parallélogramme déformable dans un plan vertical.

Une autre extrémité de la biellette 47 est articulée sur le support tracteur 45, et une seconde biellette 48 est également articulée sur l'élément tubulaire 17 et le support tracteur 45 de manière à composer avec la première biellette 47 un autre parallélogramme articulé déformable dans un plan vertical.

En fait, ces pièces sont disposées en double, de part et d'autre du bras 4.

On voit immédiatement que ces parallélogrammes articulés assurent le maintien et le parallélisme constant du support tracteur 45.

Ce dernier comporte un berceau solidaire des biellettes 47, 48 (figures 8 et 9) et qui offre un pivot 49 à une pièce 50 recevant le boîtier 51 du tracteur. La pièce 50 est libre en rotation dans un plan parallèle au bras de traction 4. Le boîtier 51 est également monté en pivotement libre sur un axe 63 horizontal transversal de la pièce 50. Le tracteur à proprement parler est classique et n'a pas besoin d'être décrit.

### 4. Le bras de contre-extension

Le bras 8 est mobile horizontalement autour du pivot 7. Son blocage et son déblocage sont commandés par une poignée 52 agissant par l'intermédiaire d'un arbre de commande sur un mors de blocage venant porter sur le pivot 7.

Le bras 8 porte un support coulissant 53 pouvant être bloqué en toute position sur la longueur du bras 8. Une tige coulissante verticale 54 est montée sur le support 53, de façon réglable en hauteur et en angulation dans un plan horizontal grâce à une vis de blocage 55. Cette tige 54 supporte le boîtier 56 du tracteur de contre-extension, lequel comporte un dispositif d'angulation dans un plan vertical, analogue à celui du tracteur du bras de traction.

### 5. Le support pelvien

Le support pelvien 6 est articulé sur un pivot vertical 5 dans le bâti 1.

La couronne 11 et la colonne 12 viennent coiffer une broche verticale 57 pourvue d'encoches d'indexage 58 et portée par un bras horizontal 59 solidaire du pivot 5.

Sur la face inférieure du bras 59, un ergot 60 peut venir s'insérer dans l'un ou l'autre des différents crans 61, formés sur la face supérieure du bâti 1.

Pour placer le support pelvien d'une position latérale à une autre, il suffit de dégager l'ergot 60 des crans 61 (figure 11) et de faire pivoter le bras 59 autour du pivot 5, puis d'engager à nouveau l'ergot 60 dans le cran 61 choisi. Un épaulement 62 est prévu pour que le bras 59 vienne buter contre le bâti 1 à une hauteur compatible avec l'enfoncement de l'ergot 60 dans un cran 61. Il reste ensuite à réorienter la couronne 11 par pivotement autour de la broche 57.

## Revendications

1. Dispositif de traction pour tables d'opération d'appareillages de chirurgie orthopédique, comprenant :
   - un bâti (1) définissant un plan médian vertical (10),
   - un premier et un second bras (4, 8) articulés sur le bâti (1) de manière à être mobiles au moins horizontalement, les bras étant dotés de moyens de blocage (13, 15) dans leur position et de moyens de traction (14, 16), et
   - un support pelvien (6) lié au bâti (1) et réglable latéralement par rapport audit plan médian (10), caractérisé en ce que les deux bras (4, 8) sont articulés dans ledit plan médian (10), le premier bras (4) étant un bras central de traction articulé de manière à être mobile horizontalement et verticalement, le second bras (8) étant un bras central de contre-extension, en ce que des moyens sont prévus pour monter le premier bras (4) sur le bâti de manière à présenter à la fois un mouvement horizontal autour d'un premier axe (19) dans ledit plan médian (10) et un mouvement vertical autour d'un second axe (30) perpendiculaire audit plan médian (10) et en ce que des moyens sont prévus pour monter le second bras (8) sur le bâti pour permettre exclusivement un mouvement horizontal autour d'un troisième axe (9) dans ledit plan médian (10).

2. Dispositif selon la revendication 1, caractérisé en ce que le bras (4) comporte un élément tubulaire articulé sur le bâti par un cardan (18-24).

3. Dispositif selon l'une quelconque des revendication 1 ou 2, caractérisé en ce que les moyens de blocage (15) du bras central (4) comportent un embout de serrage (34) coaxial au bras et manoeuvrable en rotation, traversant des lumières méridiennes croisées (27, 29) de deux fuseaux sphériques (26, 28) articulés sur des axes orthogonaux, l'embout (34) comportant deux filetages inversés coopérant avec des mors (37, 39) mobiles en translation sur l'embout (34) et situés de part et d'autre du croisement des fuseaux de manière à entraîner par leur rapprochement ou leur éloignement mutuel, le blocage par friction ou le déblocage du bras.

4. Dispositif selon la revendication 3, caractérisé en ce qu'un patin de contact (38) est interposé entre les fuseaux (26, 28) au niveau de leur croisement.

5. Dispositif selon l'une quelconque des revendications 3 ou 4, caractérisé en ce que l'embout de serrage est manoeuvrable grâce à la rotation d'une poignée (25), située en bout de bras (4), transmise par un arbre de commande (33).

6. Dispositif selon l'une quelconque des revendications 1 à 5, caractérisé en ce que le bras de traction (4) comporte un élément tubulaire (17) associé à des bielles (46) et biellettes (47, 48) de manière à former des parallélogrammes articulés maintenant en permanence le parallélisme avec le bras (4) du support tracteur (45) des moyens de traction (16).

7. Dispositif selon l'une quelconque des revendications 1 à 6, caractérisé en ce que le bras de traction (4) comporte des ressorts pneumatiques (42) de compensation de son poids propre.

8. Dispositif selon l'une quelconque des revendications 1 à 7, caractérisé en ce que le support pelvien (6) est ajustable latéralement, grâce à un pivot (5) lié au bâti (1) et un système de crantage (60, 61).

9. Dispositif selon l'une quelconque des revendications 1 à 8, caractérisé en ce que le support pelvien (6) comprend un pivot vertical (5) qui est disposé coaxialement par rapport audit troisième axe (9).

## Ansprüche

1. Streckvorrichtung für Operationstische und Zubehör für orthopädische Chirurgie, mit :
   einem Gestell (1), das eine vertikale Mittenebene (10) definiert,
   einem ersten und einem zweiten Arm (4, 8), die an dem Gestell (1) derart angelenkt sind, dass sie horizontal beweglich sind, wobei die Arme mit Blockiereinrichtungen (13, 15) in ihrer Stellung und mit Streckeinrichtungen (14, 16) versehen sind, und
   einer Beckenstütze (6), die mit dem Gestell (1) verbunden und seitlich bezüglich der Mittenebene (10) einstellbar ist, **dadurch gekennzeichnet, dass** die beiden Arme (4, 8) in der genannten Mittenebene (10) angelenkt sind, wobei der erste Arm (4) ein zentraler Streckarm ist, der derart angelenkt ist, dass er horizontal und vertikal bewegbar ist, der zweite Arm (8) ein Zentralarm der Gegenausdehnung ist, dass Einrichtungen vorgesehen sind, um den ersten Arm (4) auf dem Gestell derart anzubringen, dass er gleichzeitig eine horizontale Bewegung um eine erste Achse (19) in der Mittenebene (10) und eine Vertikalbewegung um eine zweite Achse (30) senkrecht zur Mittenebene (10) ausführen kann, und dass Einrichtungen vorgesehen sind, um den zweiten Arm (8) an dem Gestell anzubringen, um ausschliesslich eine horizontale Bewegung um eine dritte Achse (9) in der Mittenebene (10) zu erlauben.

2. Vorrichtung nach Anspruch 1, **dadurch gekennzeichnet**, dass der Arm (4) ein rohrförmiges Element aufweist, das an dem Gestell durch ein Kardanelement (18-24) angelenkt ist.

3. Vorrichtung nach Anspruch 1 oder 2, **dadurch gekennzeichnet**, dass die Blockiereinrichtung (15) des Mittenarms (4) ein Klemmendstück (34) aufweist, das koaxial zum Arm verläuft und drehbar ist, welches gekreuzte Meridianöffnungen (27, 29) von zwei Kugelzweiecken (26, 28) durchquert, die auf senkrechten Achsen angelenkt sind, wobei das Endstück (34) zwei entgegengerichtete Gewinde aufweist, die mit Spannbacken (37, 39) zusammenwirken, die längsbeweglich auf dem Endstück (34) sind und beiderseits der Kreuzung der Kugelzweiecke derart angeordnet sind, dass sie durch ihr gegenseitiges Annähern oder Entfernen das Blockieren durch Reibung oder das Lockern des Armes hervorrufen.

4. Vorrichtung nach Anspruch 3, **dadurch gekennzeichnet**, dass ein Kontaktschuh (38) zwischen die Zweiecke (26, 28) auf der Höhe ihres Kreuzens gesetzt ist.

5. Vorrichtung nach Anspruch 3 oder 4, **dadurch gekennzeichnet**, dass das Klemmendstück dank der Drehung eines Griffstücks (25) betätigbar ist, das am Ende des Armes (4) angeordnet ist, übertragen durch eine Steuerwelle (33).

6. Vorrichtung nach einem der Ansprüche 1 bis 5, **dadurch gekennzeichnet**, dass der Streckarm (4) ein rohrförmiges Element (17) aufweist, das Gliedern (46) und Schwingarmen (47, 48) derart zugeordnet ist, dass gelenkige Parallelogramme gebildet werden, die ständig die Parallelität mit dem Arm (4) der Streckenstütze (45) der Streckeinrichtung (16) halten.

7. Vorrichtung nach einem der Ansprüche 1 bis 6, **dadurch gekennzeichnet**, dass der Streckarm (4) pneumatische Federn (42) zur Kompensation seines Eigengewichts aufweist.

8. Vorrichtung nach einem der Ansprüche 1 bis 7, **dadurch gekennzeichnet**, dass die Beckenstütze (6) seitlich einstellbar ist dank eines mit dem Gestell (1) verbundenen Drehbolzens (5) und eines Einrastsystems (60, 61).

9. Vorrichtung nach einem der Ansprüche 1 bis 8, **dadurch gekennzeichnet**, dass die Beckenstütze (6) einen vertikalen Drehbolzen (5) aufweist, der koaxial bezüglich der dritten Achse (9) angeordnet ist.

**Claims**

1. Traction device for operating tables and orthopaedic surgery installations, comprising :
   - a frame (1) defining a vertical median plane (10),
   - first and second arms (4, 8) articulated on the frame (1) in such a way as to be at least horizontally mobile, the arms being equipped with means (13, 15) for locking them in their position and with traction means (14, 16), and
   - a pelvis support (6) connected to the frame (1) and adjustable laterally with respect to said median plane (10), characterized in that the two arms (4, 8) are articulated in said median plane (10) so as to be horizontally and vertically mobile, the second arm (8) being a central counter-extension arm, in that means are provided for mounting the first arm (4) on the frame in such a way as to present both a horizontal movement about a first axis (19) inside said median plane (10) and a vertical movement about a second axis (30) perpendicular to said median plane (10) and in that means are provided for mounting the second arm (8) on the frame in order to allow exclusively a horizontal movement about a third axis (9) inside said median plane (10).

2. Device according to claim 1, characterized in that the arm (4) comprises a tubular element articulated on the frame via a universal joint (18-24).

3. Device according to any one of claims 1 and 2 characterized in that the means (15) for locking the central arm (4) comprise a clamping stud (34) coaxial to the arm and adapted to be operated in rotation which stud passes through crossed meridian slots (27, 29) in two spherical lunes (26, 28) articulated on orthogonal axes, the stud (34) comprising two inverted threads cooperating with jaws (37, 39) mobile in translation on the stud (34) and situated on either side of the crossing of the lunes so as to cause by mutual approach or moving apart, the locking by friction or unlocking of the arm.

4. Device according to claim 3, characterized in that a contact shoe (38) is interposed between the lunes (26, 28) at the crossing thereof.

5. Device according to any one of claims 3 and 4, characterized in that the clamping stud is manoeuvrable thanks to the rotation of a handle 25 located at the end of the arm 4, transmitted by a control shaft 33.

6. Device according to any one of claims 1 to 5, characterized in that the traction arm (4) comprises a tubular element (17) associated with connecting rods (46) and bars (47, 48) so as to form articulated parallelograms for permanently maintaining the parallelism with the arm (4) of the tractor support (45) of the traction means (16).

7. Device according to any one of claims 1 to 6, characterized in that the traction arm (4) comprises pneumatic springs (42) for compensating its own weight.

8. Device according to any one of claims 1 to 7, characterized in that the pelvis support (6) is laterally adjustable, due to a pivot (5) connected to the frame (1) and a catching system (60, 61).

9. Device according to any one of claims 1 to 8, characterized in that the pelvis support (6) comprises a vertical pivot (5) which is disposed coaxially with respect to said third axis (9).

**Fig.1**

Fig.2

Fig.3

EP 0 240 430 B1

Fig.4

Fig.5

10

Fig.6

Fig.7

Fig.8

51

50

45

48

47

48

47

50

51

49

45

63

48

47

Fig.9

Fig.10

Fig.11

Fig.12